Europäisches Patentamt

European Patent Office

Office européen des brevets

(11) Publication number: **0 068 736**
**B1**

(12) **EUROPEAN PATENT SPECIFICATION**

(45) Date of publication of patent specification: **11.09.85**

(21) Application number: **82303151.3**

(22) Date of filing: **17.06.82**

(51) Int. Cl.⁴: **C 07 B 57/00, C 07 C 51/487, C 07 C 61/37**

(54) Resolution of d,l-trans-chrysanthemic acid L-(-)-ephedrine.

(30) Priority: **18.06.81 US 274841**

(43) Date of publication of application:
**05.01.83 Bulletin 83/01**

(45) Publication of the grant of the patent:
**11.09.85 Bulletin 85/37**

(84) Designated Contracting States:
**CH DE FR GB IT LI NL**

(56) References cited:
**GB-A-2 047 696**
**US-A-4 257 976**

(73) Proprietor: **STAUFFER CHEMICAL COMPANY**
**Westport Connecticut 06880 (US)**

(72) Inventor: **Rumanowski, Edmund Joseph**
**57 Curtis Street**
**Dover New Jersey 07801 (US)**

(74) Representative: **Smith, Sydney et al**
**Elkington and Fife High Holborn House 52/54**
**High Holborn**
**London WC1V 6SH (GB)**

Courier Press, Leamington Spa, England.

### Description

The present invention relates to the resolution of d,l-trans-chrysanthemic acid. More particularly, the present invention relates to the resolution of d,l-trans-chrysanthemic acid, using L-(−)-Ephedrine as the resolving agent.

One of the most well-known classes of natural insecticides for domestic use are those known as pyrethrines, which are extracted from pyrethrum flowers. These compounds are known to be highly effective insecticides, yet they are characterized by extremely low mammalian toxicity.

Due to the difficulties involved in producing these compounds naturally, a great deal of effort has been made to develop synthetic analogues, and these efforts have been successful.

The synthetic analogues which have been prepared are, for the most part, esters of chrysanthemic acid. Chrysanthemic acid, on the other hand, occurs in four different forms due to double isomerization on the molecule, optical and geometrical. One of these isomes, the d-trans isomer, is much more desirable than the others, because it results in insecticides which are much more active than those which are prepared from the other isomers.

Although the cis- and trans-isomers can be separated by any of several well-known techniques, the resolution of the d-trans and l-trans isomers presents more difficulty. Several methods have been developed for the resolution of the d- and l-transisomers from mixtures thereof, but each of them are characterized by undesirable features.

Thus, for example, U.S. Patent 3,666,798 teaches the resolution of d and l-chrysanthemic acid using L-lysine as the resolving agent. In accordance with the patent the chrysanthemic acid to be resolved is heated with L-lysine in a solvent, the mixture is then cooled to crystallize d-chrysanthemic acid L-lysine salt, and the salt is then decomposed with a dilute mineral acid. This process, however, is less than desirable because the L-lysine is not generally available as a commercial product.

U.S. Patent 3,842,125 teaches a process wherein (−)-α-(1-naphthyl)-ethylamine is used as a resolving agent. Unfortunately, however, this resolving agent is a specialty chemical and very expensive.

A process has been found which eliminates many of the prior art difficulties and enables the resolution of d-trans-chrysanthemic acid to be achieved in relatively high yields and at a reasonable cost.

It has now been found that d,l-trans-chrysanthemic acid can be effectively resolved in high yields by using L-(−)-Ephedrine as the resolving agent. The reaction is conducted in the presence of water.

In accordance with the present invention, there is provided a process for the isolation of d-trans-chrysanthemic acid from a mixture of d-trans-chrysanthemic acid and l-trans-chrysanthemic acid which comprises reacting the mixture with L-(−)-Ephedrine in an organic solvent, in the presence of water, cooling the resulting reaction mixture to form crystals of L-(−)-Ephedrine-d-transcrysanthemic acid salt, decomposing the salt and recovering therefrom the d-trans-chrysanthemic acid.

While the process of the present invention produces a high yield of relatively pure d-trans-chrysanthemic acid, the product may be recrystallized to improve purity even further.

The L-Ephedrine which is used as the resolving agent in the process of the present invention is an item of commerce and is readily available from many sources of supply.

The solvents which are used in the practice of the present invention are those in which the Ephedrin salt of l-trans-chrysanthemic acid is soluble, but in which the salt of d-trans-chrysanthemic acid is insoluble. These solvents include hexane, cyclohexane, toluene, heptane and mixtures thereof; although heptane is preferred. It is also preferred that the solvent be used in an amount which will result in an essentially saturated solution, because that will facilitate the formation of the salt crystals.

The presence of water in the reaction mixture enables one to produce consistently high yields of high quality product.

Prior to the discovery of the advantages provided by the presence of water in the process, the experiments produced inconsistent results. The difficulties observed have been confirmed by the failure of other workers in their attempts to use ephedrine as a resolving agent for chrysanthemic acid, as reported in an article by Campbell and Harper in J. Sci. Food Agric., 3, April, 1952 (page 190).

It appears that the inconsistencies in the results obtained initially were due to a variation in the amount of water present during the process. The presence of water enables consistent and superior results to be obtained.

The amount of water used is very small. For example, in a preparation involving an initial charge of 3.4 grams of trans-chrysanthemic acid and 1.7 grams of l-ephedrine, added four drops, or approximately 0.1 gram of water were added; and in another preparation where 10.2 grams of trans-chrysanthemic acid and 5.1 grams 1-ephedrine were used, 0.3 grams of water were employed. In both cases yields of 67% of theoretical were obtained.

While a minimum threshold amount of water which should be present has not been determined, the actual amount added generally ranges from 1 to 2 moles water per mole ephedrine, with the upper end of this range being preferred because it generally results in greater yields. In a particularly preferred embodiment, water is added until the solubility limit of the reaction mixture for water is approximately reached or even slightly exceeded. It will, of course, be understood by those skilled in the art that excessive quantities of water should not be added, because the salts being formed would be soluble in the water. These limitations are, however, intended to indicate the preferred embodiments and do not constitute limitations on the scope of the invention.

In practicing the process of the present invention, L-(−)-Ephedrine and the d,l-trans-chrysanthemic acid in the ratios of approximately 1/2 mole L-(−)-Ephedrine per mole d,l-trans-chrysanthemic acid, are brought together in a solvent to which water has been added. The amount of solvent used should be relatively small, so that a highly concentrated or even saturated solution will result, which facilitates the formation of the final product crystals. A reactant concentration of from 14—18% by weight of solvent is typical, but by no means limiting.

The initial reaction mixture may be formed at room temperatures, but it is preferred to heat the mixture somewhat, with stirring, to accelerate solution of the reactants in the solvent, and to also accelerate the reaction rate. Preferred temperature range from 40°C to 60°C. Once the reactants have dissolved the reaction mixture can be cooled to crystallize out the L-(−)-Ephedrine salt of the d-trans-chrysanthemic acid. The salt crystals can then be decomposed with either an acid, such as HCl or $H_2SO_4$; or a base, such as NaOH or KOH to regenerate the d-trans-chrysanthemic acid. The L-(−)-Ephedrine which is also regenerated can be recovered and reused.

In order that the present invention be more fully understood, the following examples are given by way of illustration. All parts and percentages are by weight unless otherwise specifically designated. Examples 1 and 2 are comparative.

Example 1

L-Ephedrine in the amount of 3.3 grams, d,l-trans-chrysanthemic acid in the amount of 3.4 grams and 40 grams of hexane were mixed and heated to 45—50°C in a beaker until a solution was formed. The solution was cooled with seeding in a refrigerator for one hour, resulting in the formation of a slurry. The slurry was filtered, and the solid material which was recovered was dried.

The dried product weighed 2.8 grams, representing an 85% yield; but had a melting point of 81—93°C, as compared to a melting point of 112—113.5°C for authentic L - Ephedrine - d - trans - chrysanthemic acid salt.

This material was recrystallized from 65 grams hexane, to produce 1.4 grams product, which represented a 42.5% yield (based on original charge). The recrystallized product had a melting point of 100—103.5°C, indicating that the purity had improved.

The recrystallized product was then mixed with 15 milliliters of 2N hydrochloric acid, and the resulting mixture was then extracted with 30 milliliters of a 1:1 hexane:methylene chloride solvent. The solvent was then recovered and stripped to 45°C and 2 millimeters mercury vacuum to produce a brownish oil which had a specific rotation $[\alpha]_{Na}^{24}$ of 15°.

This compares to a specific rotation of 14.4° for authentic d-trans-chrysanthemic acid.

This Example shows that the resolution process carried out in the absence of water is operable without the benefit of added water. The results, however, are not as good as those achieved with added water, according to the invention as will be shown in Examples 3 and 4.

Example 2

The experiment described in Example 1 was repeated with a different batch of L-Ephedrine. The product recovered had a melting point of 60—70°C, which was not significantly improved by subsequent recrystallization.

This demonstrates the inconsistency of the process when carried out without the benefit of added water.

Example 3

L-Ephedrine in the amount of 1.7 grams, water in the amount of 0.1 grams, d,l-trans-chrysanthemic acid in the amount of 3.4 grams and 40 grams of heptane were heated together in a beaker until everything but some excess water went into solution. The beaker contents were then cooled with seeding to 12°C, resulting in the formation of a slurry. The slurry was filtered, and the solid material which was recovered was dried at 80°C for 15 minutes. The dried product weighed 1.9 grams, representing a yield of 57.6% by weight, and had a melting point of 112.5—113.5°C, which compares favourably to the 112—113.5°C melting point of authentic L - Ephedrine - d - trans - chrysanthemic acid salt.

Example 4

L-Ephedrine in the amount of 5.2 grams, water in the amount of 0.3 grams, d,l-trans-chrysanthemic acid in the amount of 10.2 grams and 120 grams of heptane were heated together in a beaker until everything but some excess water went into solution. The beaker contents were cooled with seeding to 10°C resulting in the formation of a slurry. The slurry was filtered, and the recovered solid material was washed with low-boiling petroleum ether, and dried at 80°C for 0.5 hours. The dried material weighed 5.7 grams, representing a yield of 57.6% and had a melting point of 111.5—113°C.

Examples 3 and 4 show that when the reaction is conducted in the presence of water the process of the present invention results in the production of high yields of the L-Ephedrine salt of d-trans-chrysanthemic acid and consistently produces a high-quality product.

Example 5

The products prepared in Examples 1 and 2 were combined, and 6.7 grams of the combined product was mixed wtih 80 milliliters of 3N hydrochloric acid for 5 minutes. The mixture was then extracted twice with 30 milliliters each time of a 1:1 hexane:methylene chloride solvent. The resulting organic phases were combined and stripped to a 90°C pot temperature at a vacuum of 15 millimeters mercury. The residue consisted of 3.3 grams of an oil. The specific rotation of the oil was determined to be 14.4° which compares favorably to the literature value of 14.4° for d-trans-chrysanthemic acid.

This demonstrates that the process of the present invention can result in the production of very pure d-trans-chrysanthemic acid.

Example 6

L-Ephedrine in the amount of 16.6 grams and water in the amount of 3.6 grams were combined and heated on a steam bath until a melt was formed. Normal heptane, in the amount of 600 milliliters was then added, and the mixture was heated to 60°C to form a clear solution. D,l-trans-chrysanthemic acid, in the amount of 33.6 grams was stirred in until it too went into solution. The solution was then cooled, with seeding at 40°C to 10°C. The resulting slurry was filtered to yield 40 grams of wet solids. The wet solid product was dried in an 80°C oven for 1 hour to yield 25 grams of a dry product, representing 75% of theoretical yield, which had a melting point of 110—112°C.

This Example demonstrates that the process of the present invention is capable of producing very high yields of a pure product.

**Claims**

1. A process for the isolation of d-trans-chrysanthemic acid from a mixture of d-trans-chrysanthemic acid and l-trans-chrysanthemic acid which comprises reacting said mixture with L-(−)-Ephedrine in an organic solvent, in the presence of water, cooling the resulting reaction mixture to form crystals of L - (−) - Ephedrine - d - trans - chrysanthemic acid salt, decomposing said salt and recovering therefrom the d-trans-chrysanthemic acid.

2. A process as claimed in claim 1 characterised in that the organic solvent is a solvent selected from hexane, cyclohexane, toluene, heptane and mixtures thereof.

3. A process as claimed in claim 1 characterised in that said organic solvent is heptane.

4. A process as claimed in any of claims 1 to 3 characterised in that the amount of water present is approximately the amount required to reach the solubility limit of the resulting reaction mixture for water.

5. A process as claimed in any of claims 1 to 4 characterised in that the salt is decomposed by reacting it with an acid.

6. A process as claimed in claim 5 characterised in that the acid is hydrochloric acid or sulfuric acid.

7. A process as claimed in any of claims 1 to 4 characterised in that the salt is decomposed by reacting it with a base.

8. A process as claimed in claim 7 characterised in that the base is sodium hydroxide or potassium hydroxide.

**Revendications**

1. Procédé de séparation de l'acide d - trans - chrysanthémique à partir d'un mélange d'acide d - trans - chrysanthémique et d'acide l - trans - chrysanthémique qui consiste à faire réagir ce mélange avec de la L-(−)-éphédrine dans un solvant organique en présence d'eau, à refroidir le mélange réactionnel qui en résulte pour former des cristaux du sel de l'acide L - (−) - éphédrine - d - trans - chrysanthémique, à décomposer ce sel et à en récupérer l'acide d-trans-chrysanthémique.

2. Procédé selon la revendication 1, caractérisé en ce que le solvant organique est un solvant choisi parmi l'hexane, le cyclohexane, le toluène, l'heptane et leurs mélanges.

3. Procédé selon la revendication 1, caractérisé en ce que ce solvant organique est l'heptane.

4. Procédé selon l'une quelconque des revendications 1 à 3, caractérisé en ce que la quantité d'eau présente est approximativement la quantité requise pour parvenir à la limite de solubilité du mélange réactionnel qui en résulte pour l'eau.

5. Procédé selon l'une quelconque des revendications 1 à 4, caractérisé en ce que l'on décompose le sel en le faisant réagir avec un acide.

6. Procédé selon la revendication 5, caractérisé en ce que cet acide est l'acide chlorhydrique ou l'acide sulfurique.

7. Procédé selon l'une quelconque des revendications 1 à 4, caractérisé en ce que l'on décompose le sel en le faisant réagir avec une base.

8. Procédé selon la revendication 7, caractérisé en ce que cette base est l'hydroxyde de sodium ou l'hydroxyde de potassium.

**Patentansprüche**

1. Verfahren zur Isolierung von d - trans - Chrysanthemumsäure aus einem Gemisch von d - trans - Chrysanthemumsäure und l - trans - Chrysanthemumsäure, dadurch gekennzeichnet, daß man das genannte Gemisch mit L - (−) - Ephedrin in einem organischen Lösungsmittel in Gegenwart von Wasser umsetzt, das resultierende Reaktionsgemisch abkühlt, um Kristalle des L - (−) - Ephedrin - d - trans - Chrysanthemumsäure - Salzes zu bilden, das genannte Salz zersetzt und daraus die d - trans - Chrysanthemumsäure gewinnt.

2. Verfahren nach Anspruch 1, dadurch gekennzeichnet, daß das organische Lösungsmittel ein Lösungsmittel aus der Gruppe Hexan, Cyclohexan, Toluol, Heptan und Gemische davon ist.

3. Verfahren nach Anspruch 1, dadurch gekennzeichnet, daß das organische Lösungsmittel Heptan ist.

4. Verfahren nach einem der Ansprüche 1 bis 3, dadurch gekennzeichnet, daß die vorhandene Wassermenge ungefähr diejenige Menge ist, die erforderlich ist, um die Löslichkeitsgrenze des resultierenden Reaktionsgemisches für Wasser zu erreichen.

5. Verfahren nach einem der Ansprüche 1 bis 4, dadurch gekennzeichnet, daß man das Salz durch Umsetzung mit einer Säure zersetzt.

6. Verfahren nach Anspruch 5, dadurch gekennzeichnet, daß die Säure Salzsäure oder Schwefelsäure ist.

7. Verfahren nach einem der Ansprüche 1 bis 4, dadurch gekennzeichnet, daß man das Salz durch Umsetzung mit einer Base zersetzt.

8. Verfahren nach Anspruch 7, dadurch gekennzeichnet, daß die Base Natriumhydroxid oder Kaliumhydroxid ist.